# EUROPÄISCHE PATENTSCHRIFT

(11) **EP 1 083 821 B1**
(45) Veröffentlichungstag und Bekanntmachung des Hinweises auf die Patenterteilung: **05.08.2009**
(21) Anmeldenummer: 00922561.6
(22) Anmeldetag: 29.03.2000
(51) Int. Cl.: A61B 1/12, A61B 1/005, A61L 2/26

(54) **SCHUTZSCHLAUCH ZUR VERWENDUNG BEI DER STERILISIERUNG VON FLEXIBLEN ENDOSKOPEN**
PROTECTIVE TUBE USED IN THE STERILIZATION OF FLEXIBLE ENDOSCOPES
GAINE PROTECTRICE UTILISEE LORS DE LA STERILISATION D'ENDOSCOPES SOUPLES

(30) Priorität: 08.04.1999 DE 19915812
(43) Veröffentlichungstag der Anmeldung: 21.03.2001
(73) Patentinhaber: Karl Storz GmbH & Co. KG, 78532 Tuttlingen (DE)
(72) Erfinder: STAUD, Ralf, D-78576 Emmingen (DE)
(74) Vertreter: Weller, Wolfgang
(86) Internationale Anmeldenummer: PCT/EP2000/002744
(87) Internationale Veröffentlichungsnummer: WO 2000/060999

(56) Entgegenhaltungen:
- EP-A- 0 027 185
- DE-A- 2 140 806
- DE-A- 19 749 687

## Beschreibung

Die Erfindung betrifft ein flexibles Endoskop mit einem Schutzschlauch zur Verwendung bei der Sterilisierung von flexiblen Endoskopen in Unterdruckverfahren.

Ein derartiges Endoskop und ein Unterdruckverfahren zur Sterilisierung eines derartigen Endoskopes ist in der DE-A-197 49 687 beschrieben.

Aus der EP-A-0 027 185 ist ein flexibles Endoskop beschrieben, dessen flexible Hülle im distalen Endabschnitt durch besondere Schutzmaßnahmen gegenüber Verschleißerscheinungen beim Einsatz, insbesondere bei höheren Temperaturen, geschützt ist.

Flexible Endoskope unterscheiden sich von starren Endoskopen dadurch, daß der Schaft bei einem flexiblen Endoskop eine solche Flexibilität aufweist, daß er eine gebogene, eine stark gekrümmte oder sogar eine schlaufenförmige Form einnehmen kann. Ist das flexible Endoskop als Operations- oder Untersuchungsendoskop ausgebildet, wird es insbesondere für den Magen- und Darmbereich eingesetzt; ein solches flexibles Operationsendoskop ist aus der DE 39 28 532 A1 bekannt.

Ein weiterer verbreiteter Einsatzbereich für flexible Endoskope ist die Tiermedizin, wobei diese bspw. zur Untersuchung der oberen und unteren Luftwege und Luftsäcke von Pferden eingesetzt werden. Diese Endoskope können auch zur Untersuchung von Magen-Darm-Ulzera und des gesamten Urogenital-Traktes bei Pferden oder anderen Tieren herangezogen werden. Die Nutzlänge des flexiblen Schaftes beträgt dabei bis zu zwei Metern.

Derartige flexible Endoskope für die Tiermedizin sind bspw. aus dem Katalog "Endoskopie in der Tiermedizin" 5. Ausgabe 1/95 der Karl Storz GmbH & Co. Tuttlingen, Deutschland, bekannt. Ein in dem Katalog auf der Seite VET-H-FIB 3 gezeigtes Universal-Fiberskop mit der Typenbezeichnung 60309 VG weist ein Kopfstück auf, von dem sich ein flexibler Schaft mit einer Länge von etwa 1 m weg erstreckt.

Ein distaler Endabschnitt weist gegenüber diesem flexiblen Schaft eine noch weiter erhöhte Beweglichkeit dahingehend auf, daß dieser distale Endabschnitt gegenüber dem flexiblen Schaft bis zu 220° hakenartig oder halbkreisbogenartig gekrümmt werden kann. Wurde der flexible Schaft bspw. über mehrere Biegungen eines Darmtraktes in eine größere Körperhöhle, bspw. in einen Magen, vorgeschoben, kann der distale Endabschnitt um etwa 220° aus der Längserstreckung des Schaftes etwa halbkreisbogenförmig herausgebogen werden und gleichzeitig noch jeweils um 90° nach links und 90° nach rechts verdreht werden, so daß letztendlich ein vollkommener Rundumblick im Bereich des distalen Endes möglich ist. Damit der Schaft in diesem distalen Endabschnitt diese Krümmungen durchführen kann, ist er mit einer besonders flexiblen Hülle versehen, die etwa das Erscheinungsbild eines Gummischlauches aufweist.

Die Hülle des flexiblen Schaftes in der übrigen Schaftlänge ist dazu relativ steif ausgebildet und hat das Erscheinungsbild eines biegsamen Kunststoffrohres.

Der Schaft ist insgesamt gesehen somit als flexibles Hohlrohr, also etwa schlauchförmig, ausgebildet, in dessen Innerem zahlreiche Kanäle verlaufen, bspw. ein Instrumentenkanal, mehrere Lichtleiterkanäle, ein Bildleiterkanal mit einem abschließenden Objektiv, ein Kanal für die Objektivreinigung/Spülung und bspw. ein Insufflationskanal. Distalseitig ist ein festes Abschlußstück vorhanden, das die distalen Enden dieser unterschiedlichen Kanäle trägt. Zwischen diesem Abschlußstück und dem langen flexiblen Schaft erstreckt sich der distale Endabschnitt mit der besonders flexiblen gummiartigen Hülle.

Eine gebräuchliche Sterilisationstechnik für solche flexiblen Operationsendoskope besteht darin, das Endoskop in einem abgeschlossenen Raum mit einem Unterdruck zu beaufschlagen, wobei gleichzeitig aggressive und mikrobiozide Medien, bspw. wasserstoffperoxidhaltige Medien, als Sterilisationsmedien eingesetzt werden.

Im praktischen Einsatz wurde festgestellt, daß die flexible Hülle im distalen Endabschnitt bei dem Unterdruck dazu neigt, aufzublähen und aufzuplatzen.

Zum Herstellen eines Druckausgleichs zwischen dem Innenraum des Schaftes und dem Unterdruck der Sterilisationsapparate ist als zusätzliches Bauteil eine Druckausgleichskappe zur Entlüftung des Innenraums des Schaftes während der Sterilisation vorgesehen, das auf einen speziellen Anschluß bspw. am Kopfende angesetzt wird, der mit dem Innenraum des Schaftes in Verbindung steht. In dem eingangs erwähnten Prospekt ist auf der Seite VET-H-FIB 5 eine solche Druckausgleichskappe mit der Typenbezeichnung 60025 E ersichtlich.

Diese Druckausgleichskappe weist ein Ventil auf, über das bei Anlegen des Unterdruckes Luft aus dem Innenraum des Schaftes austreten kann, um dadurch einen Druckausgleich zu schaffen.

Im praktischen Einsatz wurde festgestellt, daß die aggressiven Sterilisationsmedien über das Ventil in den Innenraum des Endoskopes eindringen können und dort die im flexiblen Schaft aufgenommenen Bauteile beschädigen können, insbesondere die empfindlichen optischen Vorrichtungen beeinträchtigen.

Es wurden Lösungen dahingehend gesucht, anstatt der Druckausgleichskappe einen Druckausgleichsbehälter aufzusetzen, in den die aus dem Innenraum des Endoskops durch den Unterdruck abgesaugten Luftmengen eintreten und bei der Belüftung wieder zurückströmen können, dies ist aber baulich äußerst aufwendig.

Das Vorsehen einer Druckausgleichskappe als solche ist schon aufwendig, aber deswegen notwendig, da ansonsten die Gefahr von Undichtigkeiten im Bereich des distalen Endabschnittes schon nach wenigen Sterilisationszyklen auftreten.

Es ist daher Aufgabe der vorliegenden Erfindung, Maßnahmen vorzuschlagen, die das Durchführen von Unterdrucksterilisationsverfahren erlauben und die mit konstruktiv einfachen Mitteln sicherstellen, daß die flexible Hülle des distalen Endabschnittes nicht beschädigt wird und keine Undichtigkeiten auftreten.

Erfindungsgemäß wird die Aufgabe durch ein flexibles Endoskop mit einem Schutzschlauch gelöst, dessen Durchmesser derart gewählt ist, daß der Schutzschlauch passend auf den distalen Endabschnitt aufschiebbar ist, und dessen Länge derart gewählt ist, daß dieser zumindest den Endabschnitt mit der flexiblen Hülle bedeckt, und daß die Struktur des Schutzschlauches derart gewählt ist, daß die flexible Hülle bei den Unterdruakbedingungen vor Aufblähen geschützt ist, das Sterilisationsmedium jedoch zwischen Außenseite der Hülle und aufgeschobenen Schutzschlauch treten kann.

Der Schutzschlauch ist so ausgebildet, daß er als eine Art Stützschlauch dient, der die flexible Hülle des distalen Endabschnittes vor Aufblähen und somit dann auch vor Aufplatzen schützt. Gleichzeitig ist dessen Struktur durchbrochen, also so gewählt, daß das Sterilisationsmedium zwischen den Schutzschlauch und die Außenseite der Hülle des Endabschnittes treten kann, so daß dieser Bereich ebenfalls einwandfrei sterilisiert werden kann. Durch Wahl des entsprechenden Durchmessers des Schutzschlauches kann dieser auf den distalen Endabschnitt vor dem Sterilisieren einfach aufgeschoben werden und sitzt dann auch bei den entsprechenden Handhabungen unverlierbar auf dem Endabschnitt. Durch die Wahl der entsprechenden Länge ist sichergestellt, daß über die gesamte Länge des distalen Endabschnittes, in dem dieser mit einer solchen flexiblen Hülle versehen ist, diese bei Anlegen des Unterdruckes nicht aufbläht und somit nicht platzen kann.

Somit ist durch einfache mechanische Maßnahmen ein Schutz dieses kritischen Bereiches vor Aufblähen, Aufplatzen und somit Ausbilden von Undichtigkeiten geschützt.

Ein weiterer erheblicher Vorteil besteht darin, daß der Innenraum des Endoskopes bzw. des Schaftes als von den Sterilisationsmedien abgeschlossenes System ausgebildet werden kann, somit durch Ventilöffnungen oder dgl. keine Verbindung mit der Sterilisationsmediumumgebung existiert. Dadurch ist ausgeschlossen, daß diese aggressiven Medien überhaupt in den Innenraum eintreten können. Die Auswahl der Materialien des Schutzschlauches, um einerseits eine ausreichende Festigkeit zu erzielen, andererseits aber die Medien durchtreten zu lassen, ist einfach durchzuführen und ist an die jeweiligen konstruktiven Gegebenheiten des flexiblen Endoskopes anzupassen.

Somit wird die Aufgabe vollkommen gelöst.

In einer weiteren Ausgestaltung der Erfindung ist die Struktur des Schutzschlauches als Gewebe ausgebildet.

Diese Maßnahme hat den Vorteil, daß die gewebeartige Ausbildung fertigungstechnisch sehr einfach herzustellen ist, ein stabiles, dennoch filigranes Geflecht ergibt, das ausreichend Durchtrittsöffnungen für das Sterilisationsmedium freiläßt, so daß dies flächendeckend an die Außenseite der Hülle des distalen Endabschnittes treten kann, über das der Schutzschlauch geschoben ist. Ferner sind solche Gewebestrukturen kostengünstig herzustellen, so daß ein solcher Schutzschlauch als einmal verwendbares Wegwerfteil ausgebildet werden kann.

In einer weiteren Ausgestaltung der Erfindung weist der Schutzschlauch an zumindest einem Ende eine Aufweitung auf.

Diese Maßnahme hat den Vorteil, daß das aufgeweitete Ende als Aufschubhilfe bzw. Einführhilfe zum Einführen des distalen Endabschnittes in den Schutzschlauch bzw. umgekehrt beim Aufschieben des Schutzschlauches auf den distalen Endabschnitt des flexiblen Endoskopes dient. Dadurch kann nicht nur der Schutzschlauch in einem einfachen Aufschiebvorgang aufgeschoben werden, sondern es ist auch sichergestellt, daß dieser dabei nicht beschädigt wird, sondern in voller Funktionsfähigkeit aufgebracht werden kann.

In einer weiteren Ausgestaltung der Erfindung ist das Gewebe aus schraubenlinienförmig gewundenen Fäden aufgebaut.

Diese Struktur ähnelt einem Verstärkungsgewebe, wie es in Druckschläuchen üblich ist, was zum einen fertigungstechnisch sehr einfach zu bewerkstelligen ist, andererseits einen sehr sicheren Schutz gegen Aufblähen der Hülle auch bei hohen Unterdrücken darstellt, so daß auch eine versehentliche Fehlfunktion einer Unterdrucksterilisationskammer mit übermäßigem Unterdruck sicher abgefangen werden kann, d.h. auch in diesem Fall ist die Hülle des distalen Endabschnittes vor Aufblähen oder Aufplatzen geschützt.

Der Schutzschlauch kann bspw. schon nach dem originären Herstellvorgang und bei dem erstmaligen Sterilisieren aufgeschoben werden und auf dem Endabschnitt verbleiben, was dem Benutzer anzeigt, daß dieses Endoskop sterilisiert worden ist. Dies kann ebenso bei den nachfolgenden Sterilisationszyklen durchgeführt werden, d.h., nach dem Sterilisieren kann der Schutzschlauch zunächst auf diesem Endabschnitt verbleiben, so daß auch ein zusätzlicher Schutz gegen mechanische Beeinträchtigungen vorhanden ist, und erst vor dem unmittelbaren Einsatz wird der Schutzschlauch abgezogen. Der Schutzschlauch erfüllt also nicht nur eine Funktion bei dem eigentlichen Sterilisationsvorgang, sondern auch nachfolgend eine Schutzfunktion und gleichzeitig eine Hinweisfunktion dahingehend, daß dem Benutzer angezeigt wird, daß das Endoskop auch tatsächlich sterilisiert worden ist. Daher umfaßt die Erfindung auch die Kombination eines flexiblen Endoskopes mit einem auf dessen distalen Endabschnitt aufgeschobenen Schutzschlauch.

Ein Verfahren zum Sterilisieren eines flexiblen Endoskopes in Unterdruckverfahren besteht nunmehr darin, daß auf den distalen Endabschnitt des Endoskopes ein Schutzschlauch mit den zuvor beschriebenen Gestaltungsmerkmalen aufgeschoben wird, daß mit dem Zusammenbau aus Endoskop und Schutzschlauch dann ein Unterdrucksterilisierverfahren durchgeführt wird. Je nach Wunsch des Herstellers oder Benutzers kann der Schutzschlauch sofort nach der Durchführung der Sterilisierung abgenommen werden oder aber auf dem Endoskop verbleiben und erst vor dem Einsatz abgezogen werden.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels näher beschrieben und erläutert. Es zeigen:
- Fig. 1: eine perspektivische Ansicht eines flexiblen Endos- kopes mit einem distalen Endabschnitt mit einer be- sonders flexiblen Hülle,
- Fig. 2: eine stark vergrößerte Darstellung des distalen End- abschnittes des flexiblen Endoskopes von Anspruch 1 in geradliniger Ausrichtung sowie einen Schutz- schlauch entsprechend der vorliegenden Erfindung, der auf den distalen Endabschnitt aufgeschoben wer- den soll,
- Fig. 3: eine der Fig. 2 entsprechende Darstellung des dista- len Endabschnittes des flexiblen Endoskopes, nachdem der Schutzschlauch aufgeschoben worden ist, und
- Fig. 4: eine stirnseitige Ansicht des distalen Endes des flexiblen Endoskopes von Fig. 1.

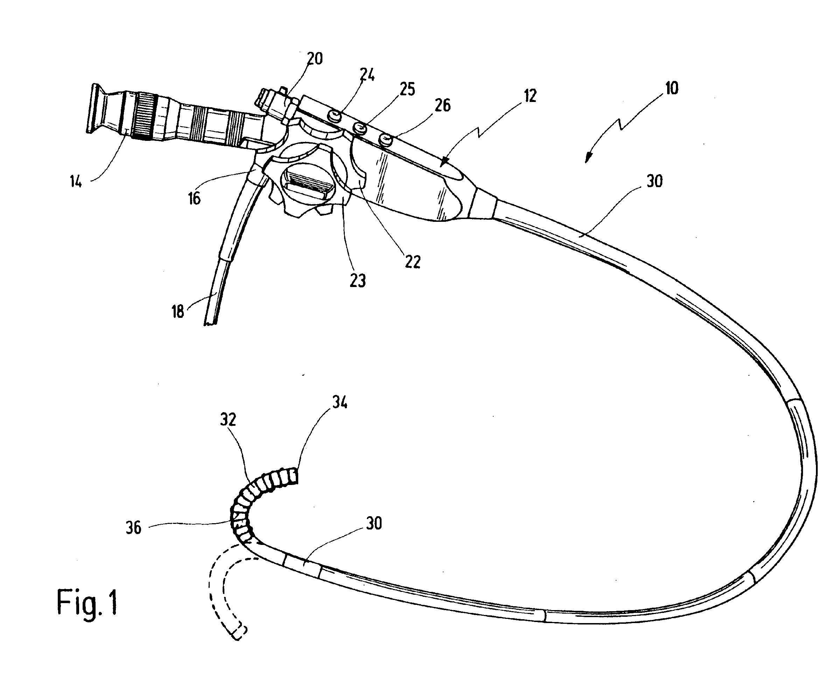

Ein in Fig. 1 dargestelltes flexibles Endoskop ist in seiner Gesamtheit mit der Bezugsziffer 10 versehen.

Das flexible Endoskop 10 weist ein in seiner Gesamtheit mit der Bezugsziffer 12 bezeichnetes Kopfstück auf.

Proximalseitig steht vom Kopfstück 12 ein Okular 14 vor. Ein seitlich vorstehender Anschluß 16 dient dazu, einen Leitungsstrang 18 anzuschließen, der die Leitungen für Beleuchtung, Spülung, Insufflation, Absaugung und dgl. aufnehmen kann. Ein weiterer, sich etwa in Richtung des Okulars 14 erstreckender Anschluß 20 ist dazu vorgesehen, daß über diesen Instrumente, bspw. Zangen, Schlingen oder dgl., in das Endoskop bzw. das Kopfstück 12 eingeschoben werden können.

Seitlich am Kopfstück 12 sind zwei Handräder 22 bzw. 23 angeordnet, deren Funktion später erläutert wird. Im Bereich des Kopfstückes 12 sind ferner Schalter 24, 25, 26 angeordnet, über die verschiedene Funktionen, wie bspw. Saugen, Spülen oder dgl., gesteuert werden können, wie das an sich bei der Ausgestaltung von flexiblen Endoskopen bekannt ist.

Vom Kopfstück 12 erstreckt sich ein langerstreckter flexibler Schaft 30 fort, der im dargestellten Ausführungsbeispiel die Länge von etwa 1 m aufweist. Der Schaft 30 ist aus einem relativ festen, jedoch flexiblen Kunststoffmaterial hergestellt, das ein Krümmen oder Biegen des Schaftes 30, wie in Fig. 1 dargestellt, ermöglicht. Ein Endabschnitt 32 weist gegenüber dem Schaft 30 noch eine erhöhte Biegsamkeit dahingehend auf, daß dieser Endabschnitt 32 noch zusätzlich um bis zu 180° etwa halbkreisförmig gebogen werden kann, wie das aus Fig. 1 ersichtlich ist. Über einen Zugseilmechanismus, der sich von einem Abschlußstück 34 bis zu den Handrädern 22 und 23 erstreckt, kann dieser Endabschnitt 32 bei an sich unveränderter Orientierung des Schaftes 30 in einer Ebene aus der in Fig. 1 ersichtlichen, mit durchgezogenen Linien dargestellten Position in eine um 180° verschwenkte, in Fig. 1 mit unterbrochenen Linien dargestellte Position verschwenkt werden. Außerdem ist es noch möglich, den gebogenen oder gekrümmten Endabschnitt 32 aus jeder Krümmungslage um etwa 90° nach links bzw. nach rechts zu bewegen, wie das an sich bekannt ist. Damit der Schaft 30 diesen extremen Krümmungsbewegungen folgen kann, ist dieser im Bereich des Endabschnittes 32 mit einer relativ weichelastischen gummiartigen Hülle 36 versehen. Diese Ausgestaltung ermöglicht, durch entsprechende Dehnungen im Bereich des äußeren längeren Krümmungsradius bzw. entsprechende Stauchungen am inneren engeren Krümmungsradius den extremen Bewegungen zu folgen.

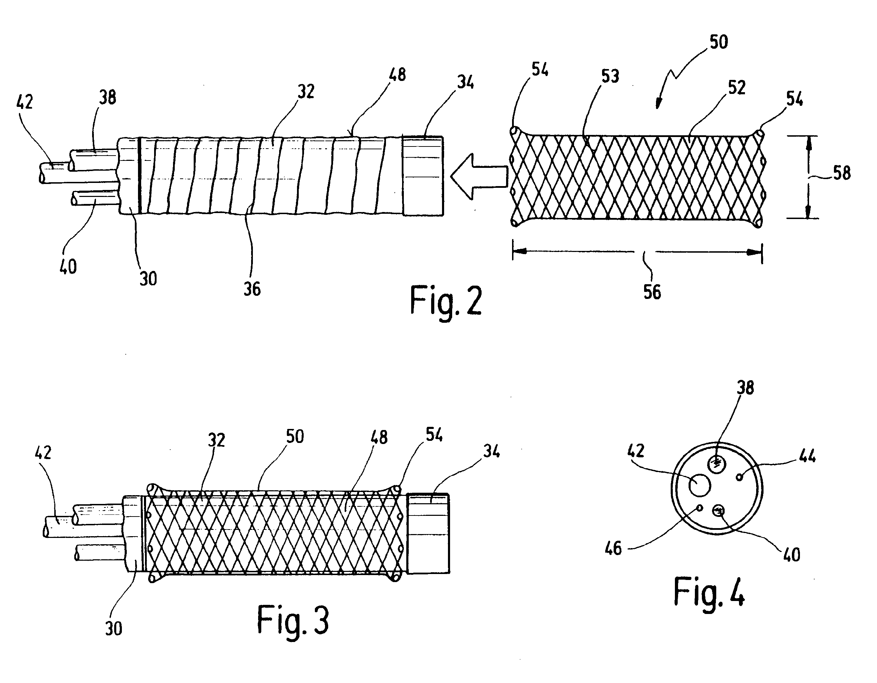

Aus der Draufsicht von Fig. 4 ist zu ersehen, daß distalseitig ein metallisches Abschlußstück 34 vorgesehen ist, an dem die entsprechenden Kanäle bzw. Bauelemente enden, also bspw. ein Bildleiter 38, ein Lichtleiter 40, ein Instrumentenkanal 42 und Bauteile des Bewegungsmechanismus, wie bspw. Seile 44 und 46.

Der Innenraum des rohrförmigen Schaftes 30 ist zur Außenseite distalseitig über das Abschlußstück 34 und proximalseitig über das Kopfstück 12 dichtend abgeschlossen.

Bei den üblichen Unterdrucksterilisationsverfahren besteht nun die Gefahr, daß der Schaft 30 im Bereich der flexiblen Hülle 36 des Endabschnittes 32 aufbläht und platzt, so daß dann Undichtigkeiten entstehen.

Dies wird nunmehr durch den in Fig. 2 dargestellten erfindungsgemäßen Schutzschlauch 50 verhindert.

Der Schutzschlauch 50 weist die Form eines Rohrabschnittes auf, dessen Struktur 52 durchbrochen ist. Dies geschieht dadurch, daß die Struktur 52, also die Rohrwand des Schutzschlauches 50, durch ein Gewebe 53 gebildet ist.

Das Gewebe 53 besteht aus gekreuzten miteinander verschlungenen schraubenlinienförmigen Fäden, die endseitig in einer trompetenförmigen Aufweitung 54 umgelenkt sind.

Die Länge 56 des Schutzschlauches 50 ist derart gewählt, daß dieser den gesamten Endabschnitt 32 des Schaftes 30 abdecken kann. Je nach Ausbildung der Länge des Endabschnittes 32 kann ein einziger oder es können auch mehrere Schutzschläuche 50 nacheinander aufgeschoben werden. Ist der Schaft 30 über seine gesamte Länge aus einem solchen weichen flexiblen Material ausgebildet, daß bei Anlegen des Unterdruckes Aufblähungen und somit ein Aufplatzen zu befürchten sind, kann selbstverständlich ein entsprechend langer oder können mehrere Schutzschläuche 50 aufgeschoben werden.

Der Durchmesser 58 des Schutzschlauches 50 ist so gewählt, daß dessen lichter Innendurchmesser in etwa dem lichten Außendurchmesser des Endabschnittes 32 entspricht.

Dadurch ist sichergestellt, insbesondere in Zusammenhang mit den trompetenartigen Aufweitungen 54, daß der Schutzschlauch 50 einfach und sicher über das Abschlußstück 34 hinweg auf den Endabschnitt 32 aufgeschoben werden kann und dort auch durch Reibschluß unverlierbar sitzt. Diese Situation ist in Fig. 3 dargestellt.

Die gewebeartige Struktur 52 sorgt für eine ausreichende Stabilität gegenüber Aufblähen der Hülle 36 bei Anlegen eines Unterdruckes, verhindert somit Aufplatzen und Undichtigkeiten des Schaftes 30. Durch den reibschlüssigen Sitz kann der Schutzschlauch 50 noch vor Einbringen des Endoskopes 10 in den entsprechenden Autoklaven auf dieses aufgeschoben werden und anschließend die entsprechenden Manipulationen durchgeführt werden, ohne daß sich dabei der Schutzschlauch 50 vom Endabschnitt 32 löst.

Durch die gewebeartige gelochte Struktur 52 ist sichergestellt, daß ein entsprechend flüssiges oder gasförmiges Sterilisationsmedium zwischen den aufgeschobenen Schutzschlauch 50 an die Außenseite 48 des Endabschnittes 32 treten kann, und zwar über die gesamte Oberfläche der Außenseite 48, d.h. auch im Bereich der Fäden des Gewebes 53. Dadurch ist dann sichergestellt, daß die gesamte Außenseite 48 mit den Sterilisationsmedien in Berührung tritt und sterilisiert wird.

Nach Durchführen des Sterilisationsvorganges kann der Schutzschlauch 50 entweder an Ort und Stelle verbleiben und erst dann abgezogen werden, wenn das flexible Endoskop 10 erneut gebraucht wird. Der Schutzschlauch 50 kann aber auch sofort abgezogen werden, je nachdem, wie das erwünscht ist. Da der Schutzschlauch 50 bei dem Sterilisationsvorgang selbstverständlich ebenfalls sterilisiert wird, kann dieser wiederverwendet werden oder auch nur zum einmaligen Gebrauch vorgesehen sein. Als Materialien, die auch gegenüber aggressiven wasserstoffperoxidhaltigen Sterilisationsmaterialien resistent sind, können Kunststoffasermaterialien, wie sie unter dem markenrechtlich geschützten Handelsnamen TREVIRA oder TYVEK bekannt sind, eingesetzt werden.

Bei dem zuvor beschriebenen Ausführungsbeispiel wurden am Schutzschlauch 50 extra trompetenartige Aufweitungen 54 vorgesehen.

Es ist auch möglich, das Abschlußstück 34 leicht konisch auszubilden, so daß dann der Schutzschlauch 50 streng geometrisch als Zylinder ausgebildet sein kann, was fertigungstechnisch einfacher zu bewerkstelligen ist, so daß dann die konische Ausbildung des Abschlußstückes 34 die Aufschub- bzw. Einführhilfe bildet.

Es wurden flexible Endoskope der Anmelderin, wie sie unter den Artikelbezeichnungen 11001BC, 11272C, 13309, 13308A bezeichnet werden, und die Schaftdurchmesser von 4 mm, 9 mm und 11 mm aufweisen, in dem flexiblen Endbereich mit einem Gewebeschlauch aus TREVIRA geschützt und bis zu 1.350 Druckwechsel durchgeführt, die den Druckwechseln bei einem Unterdrucksterilisationsverfahren entsprechen.

Auch nach dieser hohen Anzahl von Druckwechseln konnten keinerlei Undichtigkeiten im Bereich der Hülle 36 festgestellt werden, d.h. auch nach dieser hohen Anzahl an Belastungsphasen konnte nach zehn Minuten kein höherer Druckabfall festgestellt werden, als ein Druckabfall, wie er vor dieser Anzahl der Versuchszyklen beobachtet wird.

Diese Druckzyklen können dadurch simuliert werden, daß der Innenraum des Schaftes 30 mit einem solchen Überdruck beaufschlagt wird, der einem Druckunterschied zur Außenseite hin entspricht, der entsteht, wenn in einem Sterilisationsgerät ein entsprechender Unterdruck angelegt wird.

Dies demonstriert, daß durch die mechanisch einfache Maßnahme des Aufschiebens des Schutschlauches 50 mit der gewebeartigen Struktur 52 auf Dauer für Dichtigkeit im Bereich des Endabschnittes 32 gesorgt werden kann.

## Patentansprüche

1. Flexibles Endoskop mit einem Schutzschlauch zur Verwendung bei der Sterilisierung von flexiblen Endoskopen (10) in Unterdruckverfahren, wobei das flexible Endoskop (10) einen distalen Endabschnitt (32) mit einer flexiblen Hülle (36) aufweist, wobei der Durchmesser (58) des Schutzschlauches (50) derart gewählt ist, daß der Schutzschlauch (50) passend auf den distalen Endabschnitt (32) aufschiebbar ist, und die Länge (56) des Schutzschlauches (50) derart gewählt ist, daß dieser zumindest den Endabschnitt (32) mit der flexiblen Hülle (36) bedeckt, und daß die Struktur (52) des Schutzschlauches (50) derart gewählt ist, daß die flexible Hülle (36) bei den Unterdruckbedingungen vor Aufblähen geschützt ist, das Sterilisationsmedium jedoch zwischen Außenseite (48) der Hülle (36) und aufgeschobenen Schutzschlauch (50) treten kann.

2. Flexibles Endoskop nach Anspruch 1, **dadurch gekennzeichnet, daß** die Struktur (52) als Gewebe (53) ausgebildet ist.

3. Flexibles Endoskop nach Anspruch 1 oder 2, **dadurch gekennzeichnet, daß** der Schutzschlauch (50) an zumindest einem Ende mit einer Aufweitung (54) versehen ist.

4. Flexibles Endoskop nach Anspruch 2 oder 3, **dadurch gekennzeichnet, daß** das Gewebe (53) aus schraubenlinienförmig gewundenen Fäden aufgebaut ist.

5. Verfahren zum Sterilisieren eines flexiblen Endoskopes (10) in Unterdruckverfahren, mit den Schritten
- Aufschieben eines Schutzschlauches (50) nach einem der Ansprüche 1 bis 4 auf einen distalen Endabschnitt (32) des flexiblen Endoskopes (10), in dem dessen Schaft (30) eine flexible Hülle (36) aufweist,
- Durchführen eines Unterdrucksterilisierverfahrens mit dem Zusammenbau aus flexiblem Endoskop (10) und dem aufgeschobenen Schutzschlauch (50), und
- Abziehen des Schutzschlauches (50) vor einem Einsatz des Endoskopes (10).

## Claims

1. Flexible endoscope with a protective tube for use in the sterilisation of flexible endoscopes (10) in a vacuum method, wherein the flexible endoscope (10) comprises a distal end portion (32) with a flexible sleeve (36) and wherein the diameter (58) of the protective tube (50) is selected such that the protective tube (50) can be slipped onto and fits onto the distal end portion (32), the length (56) of the protective tube (50) being selected such that it covers at least the end portion (32) with the flexible sleeve (36), and wherein the structure (52) of the protective tube (55) is selected such that the flexible sleeve (36) is protected under the vacuum conditioned against swelling, where, however, the sterilisation agent reaches between the outside (48) of the sleeve (36) and the applied protective tube (50).

2. Flexible endoscope of claim 1, **characterized in that** the structure (52) is formed as a fabric (53).

3. Flexible endoscope of claims 1 or 2, **characterized in that** the protective tube (50) is provided with a widening (54) on at least one end.

4. Flexible endoscope of claims 2 or 3, **characterized in that** the fabric (53) is formed of spiral line-shaped interwined fibers.

5. Method for sterilising a flexible endoscope (10) in a vacuum method, comprising the steps of
- slipping on a protective tube (50) according to anyone of claims 1 to 4 onto a distal end portion (32) of the flexible endoscope (10) in which its shaft (30) comprises a flexible sleeve (36),
- performing a vacuum sterilisation procedure with the assembly of the flexible endoscope (10) and the applied protective tube (50), and
- removing the protective tube (50) prior using the endoscope (10).

## Revendications

1. Endoscope flexible avec une gaine protectrice destinée à être utilisée lors de la stérilisation d'endoscopes flexibles (10) dans des procédés sous vide, l'endoscope flexible (10) présentant une section terminale distale (32) avec une enveloppe flexible (36), le diamètre (58) de la gaine protectrice (50) étant choisi de façon que la gaine protectrice (50) puisse être enfilée en ajustement sur la section terminale distale (32), la longueur (56) de la gaine protectrice (50) étant choisie de façon que celle-ci recouvre au moins la section terminale (32) avec l'enveloppe flexible (36), et la structure (52) de la gaine protectrice (50) étant choisie de façon que l'enveloppe flexible (36) soit protégée contre le gonflement dans les conditions de vide, le fluide de stérilisation pouvant néanmoins passer entre le côté extérieur (48) de l'enveloppe flexible (36) et la gaine protectrice (50) enfilée.

2. Endoscope flexible selon la revendication 1, **caractérisé en ce que** la structure (52) est réalisée sous la forme d'un tissu (53).

3. Endoscope flexible selon la revendication 1 ou 2, **caractérisé en ce que** la gaine protectrice (50) est pourvue d'un élargissement (54) à au moins une extrémité.

4. Endoscope flexible selon la revendication 2 ou 3, **caractérisé en ce que** le tissu (53) est constitué de fils torsadés en hélice.

5. Procédé de stérilisation d'un endoscope flexible (10) dans des procédés sous vide, comprenant les étapes :
- enfilage d'une gaine protectrice (50) selon les revendications 1 à 4 sur une section terminale distale (32) de l'endoscope flexible (10) dont le corps (30) présente une enveloppe flexible (36),
- exécution d'un procédé de stérilisation sous vide avec l'ensemble composé de l'endoscope flexible (10) et de la gaine protectrice (50) enfilée, et
- retrait de la gaine protectrice (50) avant une utilisation de l'endoscope (10).
